Europäisches Patentamt

European Patent Office

Office européen des brevets

(19)

(11) Publication number: **0 271 143 B1**

# EUROPEAN PATENT SPECIFICATION

(12)

(45) Date of publication of patent specification: **21.08.91**  (51) Int. Cl.⁵: **C07C 7/12, C07C 11/02, C07C 2/36**

(21) Application number: **87202330.4**

(22) Date of filing: **25.11.87**

(54) Process for removing phosphine impurities from higher olefins.

<table>
<tr><td>

(30) Priority: **10.12.86 US 940386**

(43) Date of publication of application:
**15.06.88 Bulletin 88/24**

(45) Publication of the grant of the patent:
**21.08.91 Bulletin 91/34**

(84) Designated Contracting States:
**DE ES FR GB IT NL**

(56) References cited:
**EP-A- 0 125 833**
**FR-A- 2 229 440**
**GB-A- 1 161 773**
**US-A- 3 284 531**
**US-A- 4 675 463**

</td><td>

(73) Proprietor: **SHELL INTERNATIONALE RE-SEARCH MAATSCHAPPIJ B.V.**
**Carel van Bylandtlaan 30**
**NL-2596 HR Den Haag(NL)**

(72) Inventor: **Paxson, Timm Edward**
**4403 Belle Hollow Drive**
**Houston Texas 77084(US)**

(74) Representative: **Aalbers, Onno et al**
**P.O. Box 302**
**NL-2501 CH The Hague(NL)**

</td></tr>
</table>

## Description

The present invention relates to a process for the purification of higher olefins by removing phosphine impurities therefrom, particularly phosphine impurities which are residues of catalysts utilized in the preparation of said olefins.

It is known in the art to prepare higher olefins (i.e., those having a carbon number of at least six) by oligomerization of lower olefins, most commonly ethylene, over a catalyst which comprises a phosphine component. Such oligomerization processes are described, for instance, in U.S. patent specification No. 4,528,416 and other patent specifications cited therein.

Higher olefins find their principal utility as intermediates in the preparation of a very wide variety of end products. For example, olefin products having carbon numbers predominantly in the $C_6$ to $C_{20}$ range are known to be particularly useful as intermediates in the preparation of surfactants, lubricants, polymers and plasticizers. In the practice of various processes for synthesizing such end products from higher olefins, it has occasionally been observed that small amounts of residual phosphine impurities substantially interfere with the processing of the olefin or adversely influence the properties of the end product. Accordingly, a process for the effective removal of phosphine impurities from higher olefins would be highly desirable.

The present invention relates to a process for removing phosphine impurities from an olefin containing such impurities, which comprises a step to contact the olefin with a cation exchange resin in the acid form. In this context it should be noted that it is known from the prior art that an anion exchange resin can be used to eliminate phosphate values from water. In U.S. patent specification No. 3,579,322 a process is described for the removal of cations from a waste aqueous stream containing phosphate values by contacting said stream with a cation exchange resin bed and wherein the cation removal is followed by the elimination of phosphate values by contacting the cation-free stream with an anion exchange resin. In an alternative embodiment of the process, according to said patent specification the aqueous stream is directly contacted with a weak-base anion exchange resin to remove phosphate values. In Japanese patent specification No. 83017885 a process is described wherein water containing both phosphate and a magnesium salt is contacted with an ion exchange resin in the calcium form. This contact yields an ion exchange in the magnesium form, and calcium phosphate which can be removed by precipitation from the aqueous solution. In Japanese patent specification No. 78031572 a process is described for the removal of phosphoric acid ions from an aqueous solution using an ion exchange means exemplified by $Al_2O_3$, $La_2O_3$, and Group VIII metal oxides.

It has now been found that phosphine impurities can be effectively removed from higher olefin products containing such impurities by treating said higher olefin products with a particularly specified cation exchange resin, which resin is a macroreticular resin in the acid form. Strong acid resins and particularly sulphonated resins are preferred.

Therefore, the present invention relates to a process for removing phosphine impurities from a higher olefin product, which comprises contacting said higher olefin product with a macroreticular cation exchange resin in the acid form.

Although the process according to the present invention can be applied with any phosphine-containing higher olefin product, the process is particularly useful for removing phosphine impurities from higher olefin products of oligomerization processes utilizing phosphine-containing catalyst systems. Various processes for the oligomerization of lower olefins to higher olefin products, utilizing as catalysts a class of chelate complexes of metals, particularly nickel, with organophosphines, are described in U.S. patent specification Nos. 4,528,416, 4,260,844, 4,020,121, 3,825,615, 3,647,915, 3,647,914, 3,644,564, 3,737,475, 3,686,351 and 3,676,523. In said patent specifications processes are described which allow for the possiblity of phosphine compounds being introduced into higher olefins products. Oligomerization, as the term is used herein, is intended to include dimerization reactions, trimerization reactions, and higher oligomerization reactions by which ethylene, propylene, and butylene are converted to higher olefinic oligomers.

In conventional practice, when higher olefins are prepared via oligomerization of lower olefins (for instance $C_2$ to $C_4$ olefins and in particular ethylene) in the presence of a catalyst system comprising a phosphine component, it is typical for the finished olefin product to contain phosphine impurities, particularly organophosphine impurities, in quantities corresponding to a concentration of elemental phosphorus in the order of 10 to 100 ppmw (parts per million, by weight). Phosphine impurities present in a given higher olefin product of such an oligomerization process may originate from the particular phosphine compound used in forming the oligomerization catalyst system (for instance, a diarylphosphinocarboxylic acid) or, alternatively, may be in the form of one or more other phosphine compounds (for instance, a diarylalkyl phosphine) to which that particular phosphine is converted during the oligomerization reaction or during the working-up procedures for the olefin product.

Preferably, according to the process of the present invention phosphine impurities are removed which are predominantly organophosphine compounds from olefins which are predominantly acyclic aliphatic olefins and have carbon numbers which are predominantly in the range from 6 to 30. The present invention is particularly useful for removing phosphine impurities from monoolefins having carbon numbers predominantly in the range from 6 to 20.

The cation exchange resin used in the process the according to present invention is a macroreticular (macroporous, highly crosslinked) resin in the acid form, i.e. a resin having a negatively charged matrix and exchangeable hydrogen cations. The resin is suitable for the weak acid type, as exemplified by common conventional resins based on acrylic or methacrylic acid, which has been cross-linked with a difunctional monomer such as divinylbenzene. Strong acid resins are, however, preferred. Particularly useful in the process according to the present invention are the strong acid resins which are sulphonated resins wherein the exchangeable hydrogen cation is provided by the $SO_3H$ moiety. Such sulphonated, strong acid resins are exemplified by the sulphonated copolymers of styrene and divinylbenzene.

Specific examples of commercially available macroreticular sulphonated strong acid ion exchange resins include those described in the following table:

| Resin | Manufacturer | Surface Area $(m^2/g)$ | Average Pore Diameter (nm) |
|---|---|---|---|
| Amberlyst 15 | Rohm & Haas Co. | 45-55 | 26.5 |
| Amberlite 200 | Rohm & Haas Co. | 35-45 | |
| XN-1010 | Rohm & Haas Co. | 570 | 5.0 |
| XN-1005 | Rohm & Haas Co. | 130 | 17.5-21.0 |
| MSC-1-H | Dow Chemical Co. | 35 | 30.0 |
| AG-MP-50 | Bio-Rad Lab., Inc. | 30-50 | |
| Duolite C26TR | Diamond Shamrock Corp. | 15-30 | |

In the process according to the present invention, liquid olefin product containing one or more phosphine impurities is contacted with solid resin, suitably in either a batch or continuous (or semi-continuous) mode. When operating in a batch mode, the contact preferably involves agitation of a mixture of olefin product and resin for about 0.01 to 10 hours, more preferably 0.1 to 3 hours, followed by separation of the resin by conventional solid-liquid separation techniques, e.g., settling, centrifugation, filtration, or the like. The process according to the present invention is often preferably carried out in a continuous mode by passing a stream of olefin through one or more contained beds of resin, e.g., fixed beds, moving beds, or fluidized beds, at a flowrate preferably ranging from a weight hourly space velocity of 0.1 to 100 hours$^{-1}$, and more preferably from 0.5 to 10 hours$^{-1}$. The present invention is very suitably carried out in conventional contactors designed for ion exchange service.

The conditions of contacting higher olefin product with cation exchange resin are not narrowly critical to the performance of the process according to the invention. Nevertheless, preference is given to certain specified operating ranges. For instance it is preferred that the higher olefin product is contacted with the cation exchange resin at a temperature in the range from 5 to 100°C, more preferably in the range from 5 to 70°C and most preferably in the range from 10 to to 55°C. More broadly, operating temperatures are only limited by the freezing point and boiling point of the appropriate higher olefin product and the temperature stability of the resin. Process pressure is specified only in terms of a pressure sufficient to maintain the higher olefin product in the liquid phase. Atmospheric or greater pressure (e.g., 1-100 bar) and typical ambient temperatures (i.e., 10-35°C) are preferred.

After the contact step, the resin may, if desired, be regenerated for reuse, although regeneration is not a necessary requirement of the present invention. In cases in which phosphine impurities are initially present in the feedstock in a low concentration (e.g., up to 50 ppmw), the process according to the invention can in many cases be economically carried out with a "throw-away bed", i.e. without regeneration of the resin. In this regard, it has been found that for treatment of the olefin products of most interest, a bed can typically be used to treat a quantity of olefin product up to 500 times its own weight before there is a significant loss in its phosphine removal capabilities. Capacity of the resin bed for treatment of the olefin product will, of course, in any given case depend on the particular concentration of phosphine impurities in the olefin product. If regeneration of the resin bed is desired, it can be accomplished by conventional methods, involving, for instance, treatment of the resin with an acid wash (e.g., aqueous or alcoholic hydrochloric acid or acetic acid, or the like), followed by water wash to restore the hydrogen cations in the resin. Following such treatment, the resin is preferably dried to remove excess water before reuse.

It has been found that when the process of the present invention is carried out at preferred operating conditions the phosphine content of the olefins can be reduced to a level of less than 1 part per million.

The present invention is further illustrated by the following Example. Comparative experiments are also provided, showing the relatively poor phosphine removal performance of processes which are not in accordance with the present invention as common ion exchange materials are used in stead of the specified acid ion exchange resin-type.

### Example

In a process according to the present invention a stream of $C_{16}$ alpha-olefins having a predominantly linear carbon structure was contacted with a bed of a macroreticular cation exchange resin in the acid form. The alpha-olefin product stream has been prepared by oligomerization of ethylene using a phosphine-containing catalyst, and has a residual content of phosphine (primarily ethyldiphenyl phosphine) determined by chromatography to represent 30-35 ppmw of elemental phosphorus. The bed consisted of two sections of resin, through which olefin product flowed in series, and had a provision for withdrawing a sample of the olefin product between the two sections. Use of the divided, two-section, bed and a flow of olefin product in series through the two sections permitted investigation at two space velocities in each experiment. In each case the bed was operated in an upflow mode and at a temperature of 26° C.

The $C_{16}$ olefin product stream was contacted with a bed of Dowex MSC-1-H+ cation exchange resin. The MSC-1-H+ resin is a strongly acidic sulphonated macroreticular resin characterized by a surface area of 35 square meters per gram, an average pore diameter of 30 nm, and an acid density of 4.0 milliequivalents acid (H+) per gram. Prior to use, the material was washed with 5 bedweights of 0.1 N hydrochloric acid solution, then with 10 bedweights of water. Finally, the resin was dried at 100° C in a flowing nitrogen atmosphere. The Flowrate of the olefin product was adjusted to provide a weight hourly space velocity (WHSV) of 5.0 in the first of the two bed sections and a WHSV of 2.5 in the overall bed. Amounts (in parts per million by weight) of phosphorus in the effluent olefin which, were determined over the duration (in hours) of the experiment and for the number of bedweights of olefin which had been contacted with the resin (i.e., the ratio of the cumulative weight of the olefin stream to the weight of the resin with which it was contacted), are shown in Table I.

Table I

| | WHSV 5 | | WHSV 2.5 | |
|---|---|---|---|---|
| | | Elemental | | Elemental |
| | Througput | Phosphorus | Throughput | Phosphorus |
| Hours | (Bedweights) | (ppmw in effluent) | (Bedweights) | (ppmw in effluent) |
| 0 | 0.0 | | 0.0 | |
| 8 | 27.3 | 21.0 | 13.1 | 0.5 |
| 16 | 74.5 | 0.9 | 36.7 | 0.0 |
| 24 | 126.4 | 4.3 | 10.3 | 0.0 |
| 32 | 161.2 | 5.1 | 80.0 | 0.1 |
| 40 | 189.1 | 3.9 | 93.9 | 0.1 |
| 48 | 236.9 | 13.5 | 117.9 | 0.3 |
| 56 | 266.9 | 18.3 | 132.9 | 0.4 |
| 64 | 302.1 | 6.1 | 150.5 | 0.3 |
| 72 | 343.2 | 25.7 | 171.0 | 0.7 |
| 80 | 366.3 | 27.3 | 182.5 | 1.6 |
| 88 | 401.1 | 19.9 | 199.9 | 2.3 |
| 96 | 441.9 | 26.8 | 220.3 | 2.7 |
| 104 | 473.1 | 30.4 | 235.9 | 6.5 |

These results indicate a very effective removal of phosphine impurities up to cumulative olefin product contact flows of 200 bedweights, which roughly corresponds to adsorption of a basic phosphorus atom of the phosphine at each available acid ($SO_3H$) site of the resin.

Comparative Experiment A

A comparative experiment was carried out in which the $C_{16}$ olefin product stream was contacted with a bed of alumina. Apart from this substitution of ion exchange material, this experiment was carried out in a virtually analogous manner as the process described in the above Example.
The specific alumina tested was KC-300 alumina, a product of Shell Chemical Company, characterized by a surface area of 200-400 square metres per gram. The material was in the form of a 0.25 cm diameter cylindrical extrudate. The resin was dried at 525° C for 10 hours in a flowing nitrogen stream before use.
Results of Comparative Experiment A are presented in Table II:

### Table II

| | WHSV 5 | | | WHSV 2.5 | |
|---|---|---|---|---|---|
| | | Elemental | | | Elemental |
| | Throughput | Phosphorus | | Throughput | Phosphorus |
| Hours | (Bedweights) | (ppmw in effluent) | | (Bedweights) | (ppmw in effluent) |
| 0 | 0.0 | | | 0.0 | |
| 4 | 9.8 | 2.7 | | 4.9 | 0.4 |
| 8 | 29.0 | 8.5 | | 14.5 | 0.6 |
| 12 | 45.5 | 11.8 | | 22.8 | 2.6 |
| 16 | 61.5 | 12.5 | | 30.8 | 3.0 |
| 20 | 78.0 | 16.0 | | 39.0 | 2.6 |
| 24 | 96.5 | 18.6 | | 48.3 | 6.1 |
| 28 | 110.0 | 15.9 | | 55.0 | 4.8 |
| 32 | 127.0 | 24.9 | | 63.5 | 10.6 |
| 36 | 143.0 | 23.3 | | 71.5 | 4.3 |
| 40 | 161.3 | 13.2 | | 80.6 | 6.5 |
| 44 | 180.3 | 12.2 | | 90.1 | 5.8 |
| 48 | 198.8 | 13.6 | | 99.4 | 7.3 |
| 52 | 215.0 | 26.6 | | 107.5 | 14.7 |
| 56 | 233.3 | 27.0 | | 116.6 | 16.4 |
| 60 | 247.3 | 22.7 | | 123.6 | 12.4 |
| 64 | 261.8 | 14.0 | | 130.9 | 17.5 |
| 68 | 277.8 | 28.0 | | 138.9 | 19.4 |
| 72 | 291.8 | 34.2 | | 145.9 | 21.1 |
| 76 | 309.3 | 20.3 | | 154.6 | 17.2 |
| 80 | 331.3 | 26.9 | | 165.6 | 21.0 |
| 84 | 349.8 | 23.8 | | 174.9 | 0.0 |
| 88 | 365.5 | 31.3 | | 182.8 | 27.2 |

These results show that alumina is substantially less effective for the removal of phosphines from a higher olefin product than the specific ion exchange material used in the process according to the present invention. Although the alumina tested did not offer acceptable performance for removal of phosphine impurities, it was found to be effective for removal of certain oxygenated impurities from the olefin. Therefore, in a preferred embodiment of the process according to the present invention olefin product is contacted with both the specified acid resin and alumina, either in multiple beds or a single mixed bed to achieve removal of both phosphines and other impurities.

### Comparative Experiment B

A comparative experiment was carried out wherein, the $C_{16}$ olefin product stream was contacted with a bed of 13-X zeolite, a product of the Linde Division of Union Carbide Corporation. The material was in the form of a 0.16 cm diameter cylindrical extrudate. Before use, the resin was dried at 250° C for four hours in a flowing nitrogen stream. Apart from this substitution of ion exchange material, this experiment was carried out in virtually analogous manner as the process described in the above Example.

Results of Comparative Experiment B are presented in Table III:

## Table III

| | WHSV 45 | | WHSV 2.5 | |
| | | | | |
| | | Elemental | | Elemental |
| | Throughput | Phosphorus | Throughput | Phosphorus |
| Hours | (Bedweights) | (ppmw in effluent) | (Bedweights) | (ppmw in effluent) |
|---|---|---|---|---|
| 0 | 0.0 | | 0.0 | |
| 4 | 14.8 | 4.4 | 7.4 | 0.0 |
| 8 | 30.8 | 15.6 | 15.4 | 1.5 |
| 12 | 50.4 | 17.8 | 25.2 | 3.4 |
| 16 | 68.8 | 20.7 | 34.4 | 7.0 |
| 20 | 87.4 | 19.4 | 43.7 | 8.7 |
| 24 | 105.0 | 20.8 | 52.5 | 10.8 |
| 28 | 119.4 | 21.5 | 59.7 | 10.8 |
| 32 | 138.2 | 21.5 | 69.1 | 10.9 |
| 36 | 154.8 | 22.9 | 77.4 | 13.0 |
| 40 | 173.4 | 23.9 | 86.7 | 12.2 |
| 44 | 191.2 | 21.5 | 95.6 | 12.6 |
| 48 | 207.8 | 21.3 | 103.9 | 13.5 |
| 52 | 222.8 | 12.4 | 111.4 | 12.4 |
| 56 | 238.4 | 13.2 | 119.2 | 13.2 |
| 60 | 253.4 | 12.4 | 126.7 | 12.4 |
| 64 | 272.0 | 13.9 | 136.0 | 13.9 |
| 68 | 288.2 | 20.2 | 144.1 | 14.0 |
| 72 | 295.8 | 27.4 | 147.9 | 20.2 |
| 76 | 309.8 | 27.2 | 154.9 | 16.5 |
| 80 | 329.2 | 25.6 | 164.6 | 19.0 |
| 84 | 346.8 | 16.3 | 173.4 | 21.7 |
| 88 | 355.1 | 31.1 | 177.6 | 22.9 |

These results again illustrate that the use of a specified acid ion exchange resin is critical for effective removal of phosphines impurities.

Comparative Experiment C

A comparative experiment, was carried out wherein, the $C_{16}$ olefin product stream was contacted with a bed of activated carbon, specifically a 60/80 mesh (corresponding to particle diameters of between 0.18-0.25 mm) granular activated carbon manufactured by the Calgon Corp. This material was water washed and dried at 250° C prior to use. Apart from this substitution of ion exchange material, this comparative experiment was carried out in a virtually analogous manner as the process described in the above Example.

Results of Comparative Experiment C are presented in Table IV:

### Table IV

| | WHSV 5 | | WHSV 2.5 | |
|---|---|---|---|---|
| Hours | Throughput (Bedweights) | Elemental Phosphorus (ppmw in effluent) | Throughput (Bedweights) | Elemental Phosphorus (ppmw in effluent) |
| 0 | 0.0 | | 0.0 | |
| 8 | 30.3 | 0.0 | 14.5 | 4.9 |
| 16 | 80.3 | 27.3 | 39.5 | 25.2 |
| 24 | 128.5 | 32.8 | 63.7 | 26.8 |
| 32 | 161.2 | 4.0 | 80.0 | 37.0 |
| 40 | 196.5 | 38.7 | 91.6 | 16.7 |
| 48 | 242.7 | 31.3 | 120.7 | 40.9 |
| 56 | 273.3 | 35.5 | 136.1 | 37.4 |
| 64 | 310.0 | 13.0 | 154.4 | 33.2 |
| 72 | 352.0 | 27.3 | 175.4 | 37.1 |
| 80 | 376.3 | 34.7 | 187.5 | 45.7 |
| 88 | 412.0 | 39.7 | 205.4 | 34.3 |
| 96 | 454.4 | 33.4 | 226.6 | 32.1 |
| 104 | 485.6 | 34.4 | 242.2 | 29.3 |

These results show that, compared to the process according to the present invention, activated carbon is substantially less effective for the removal of phosphines from a higher olefin product.

In the above description, 'Amberlyst 15', 'Amberlite 200', 'XN-1010', 'XN-1005', 'MSC-1-H', 'AG-MP-50', 'Duolite C26TR', 'Dowex MSC-1-H+', 'KC-300' and '13-X' are trade marks.

### Claims

1. A process for removing phosphine impurities from a higher olefin product containing such phosphine impurities, which comprises contacting said higher olefin product with a macroreticular cation exchange resin in the acid form.

2. A process according to claim 1, wherein the cation exchange resin is a sulphonated cation exchange resin.

3. A process according to claim 2, wherein the sulphonated resin is a sulphonated copolymer of styrene and divinylbenzene.

4. A process according to any one of claims 1-3, wherein the higher olefin product is contacted with the macroreticular cation exchange resin at a temperature in the range from 5 to 100° C.

5. A process according to claim 4, wherein the higher olefin product is contacted with the macroreticular cation exchange resin at a temperature in the range from 5 to 70° C.

EP 0 271 143 B1

6. A process according to any one of claims 1-5, wherein the higher olefin product is contacted with the resin at a weight hourly space velocity ranging from 0.1 to 100 hr$^{-1}$ .

7. A process according to claim 6, wherein the weight hourly space velocity of the olefin higher product is in the range from 0.5 to 10 hours$^{-1}$.

8. A process according to any one of claims 1-7, wherein higher olefin product(s) are used wherein the phosphine impurities are predominantly organophosphine impurities present in the higher olefin product in a concentration of 10 to 100 parts per million by weight, based on elemental phosphorus.

9. A process according to any one of claims 1-8, wherein higher olefin product(s) are used wherein the higher olefin product predominantly comprises acyclic aliphatic mono-olefins in the carbon number range from 6 to 30.

10. A process according to claim 1, wherein the higher olefin product is prepared by oligomerizing one or more lower olefin reactants in the presence of a catalyst system comprising a chelate complex of nickel with an organophosphine.

11. A process according to claim 1, wherein the higher olefin product is contacted with alumina in addition to the macroreticular cation exchange resin in the acid form for removing oxygenated impurities as well as the phosphines.

**Revendications**

1. Un procédé pour éliminer les impuretés phosphiniques d'un produit oléfine supérieure contenant de telles impuretés phosphiniques, qui comprend la mise en contact du produit oléfine supérieure avec une résine échangeuse de cations macroréticulaire dans la forme acide.

2. Un procédé selon la revendication 1, dans lequel la résine échangeuse de cations est une résine échangeuse de cations sulfonée.

3. Un procédé selon la revendication 2, dans lequel la résine sulfonée est un copolymère sulfoné de styrène et de divinylbenzène.

4. Un procédé selon l'une quelconque des revendications 1-3, dans lequel le produit oléfine supérieure est mis en contact avec la résine échangeuse de cations macroréticulaire à une température comprise entre 5 et 100° C.

5. Un procédé selon la revendication 4, dans lequel le produit oléfine supérieure est mis en contact avec la résine échangeuse de cations macroréticulaire à une température comprise entre 5 et 70° C.

6. Un procédé selon l'une quelconque des revendications 1-5, dans lequel le produit oléfine supérieure est mis en contact avec la résine à une vitesse spatiale horaire en poids comprise entre 0,1 et 100 h$^{-1}$.

7. Un procédé selon la revendication 6, dans lequel la vitesse spatiale horaire en poids du produit oléfine supérieure est comprise entre 0,5 et 10 h$^{-1}$.

8. Un procédé selon l'une quelconque des revendications 1-7, dans lequel on utilise un ou des produits oléfines supérieures dans lesquels les impuretés phosphiniques sont principalement des impuretés organophosphines présentes dans le produit oléfine supérieure à une concentration de 10 à 100 parties par million en poids, en calculant en phosphore élémentaire.

9. Un procédé selon l'une quelconque des revendications 1-8, dans lequel on utilise un ou des produits oléfines supérieures qui comprennent principalement. des monooléfines aliphatiques acycliques ayant de 6 à 30 atomes de carbone.

10. Un procédé selon la revendication 1, dans lequel le produit oléfine supérieure est préparé par oligomérisation d'une ou plusieurs oléfines inférieures en présence d'un système catalytique compre-

9

nant un complexe chélaté du nickel avec une organophosphine.

11. Un procédé selon la revendication 1, dans lequel le produit oléfine supérieure est mis en contact avec de l'alumine en plus de la résine échangeuse de cations macroréticulaire dans la forme acide pour élimination des impuretés oxygénées aussi bien que des phosphines.

**Patentansprüche**

1. Verfahren zur Entfernung von Phosphin-Verunreinigungen aus einem höheren Olefinprodukt, enthaltend derartige Phosphin-Verunreinigungen, umfassend das Zusammenbringen des erwähnten höheren Olefinproduktes mit einem makrovernetzten Kationenaustauscherharz (mit Kanalstruktur) in Säureform.

2. Verfahren nach Anspruch 1 wobei das Kationenaustauscherharz ein sulfoniertes Kationenaustauscherharz ist.

3. Verfahren nach Anspruch 2, wobei das sulfonierte Harz ein sulfoniertes Copolymer aus Styrol und Divinylbenzol ist.

4. Verfahren nach einem der Ansprüche 1 bis 3, wobei das höhere Olefinprodukt mit dem makrovernetzten Kationenaustauscherharz bei einer Temperatur im Bereich von 5 bis 100°C zusammengebracht wird.

5. Verfahren nach Anspruch 4, wobei das höhere Olefinprodukt mit dem makrovernetzten Kationenaustauscherharz bei einer Temperatur im Bereich von 5 bis 70°C zusammengebracht wird.

6. Verfahren nach einem der Ansprüche 1 bis 5, wobei das höhere Olefinprodukt mit dem Harz mit einer Raumgeschwindigkeit (weight hourly space velocity) im Bereich von 0,1 bis 100 $h^{-1}$ zusammengebracht wird.

7. Verfahren nach Anspruch 6, wobei die Raumgeschwindigkeit des höheren Olefinprodukts im Bereich von 0,5 bis 10 $h^{-1}$ liegt.

8. Verfahren nach einem der Ansprüche 1 bis 7, wobei (ein) höhere(s) Olefinprodukt(e) angewandt wird/werden, wobei die Phosphinverunreinigungen überwiegend Organophosphin-Verunreinigungen sind, die in dem höheren Olefinprodukt in einer Konzentration von 10 bis 100 ppm (Gewicht), bezogen auf elementaren Phosphor, vorhanden sind.

9. Verfahren nach einem der Ansprüche 1 bis 8, wobei (ein) höhere(s) Olefinprodukt(e) angewandt wird/werden, wobei das höhere Olefinprodukt hauptsächlich acyclische aliphatische Monoolefine mit 6 bis 30 Kohlenstoffatomen umfaßt.

10. Verfahren nach Anspruch 1, wobei das höhere Olefinprodukt hergestellt wird durch Oligomerisieren von einem oder mehreren niederen Olefin(en) als Reaktionspartner in Gegenwart eines Katalysatorsystems, umfassend einen Chelatkomplex von Nickel mit einem Organophosphin.

11. Verfahren nach Anspruch 1, wobei das höhere Olefinprodukt zusammengebracht wird mit Tonerde neben dem makrovernetzten Kationenaustauscherharz in Säureform zur Entfernung von oxidierten Verunreinigungen sowie von Phosphinen.